Europäisches Patentamt

European Patent Office

Office européen des brevets

(19)

(11) **EP 1 200 071 B1**

(12) **FASCICULE DE BREVET EUROPEEN**

(45) Date de publication et mention
de la délivrance du brevet:
**09.04.2003 Bulletin 2003/15**

(21) Numéro de dépôt: **00958607.4**

(22) Date de dépôt: **25.07.2000**

(51) Int Cl.⁷: $A61K\ 9/50$, $A61K\ 9/20$

(86) Numéro de dépôt international:
**PCT/FR00/02132**

(87) Numéro de publication internationale:
**WO 01/006982 (01.02.2001 Gazette 2001/05)**

(54) **COMPRIMES FAIBLEMENT DOSES ET PROCEDE DE PREPARATION**

NIEDRIG DOSIERTE TABLETTEN, UND VERFAHREN ZUR HERSTELLUNG

LOW-DOSE TABLETS AND PREPARATION METHOD

(84) Etats contractants désignés:
**AT BE CH CY DE DK ES FI FR GB GR IE IT LI LU
MC NL PT SE**
Etats d'extension désignés:
**AL LT LV MK RO SI**

(30) Priorité: **26.07.1999 FR 9909653**

(43) Date de publication de la demande:
**02.05.2002 Bulletin 2002/18**

(73) Titulaire: **LABORATOIRES DES PRODUITS
ETHIQUES ETHYPHARM
78550 Houdan (FR)**

(72) Inventeurs:
• **COUARRAZE, Guy
F-78340 Les Clayes-sous-Bois (FR)**
• **LECLERC, Bernard
F-91430 Igny (FR)**
• **TCHORELOFF, Pierre
F-91440 Bures sur Yvette (FR)**
• **SANIAL, Patrick
F-78630 Morainvilliers (FR)**

(74) Mandataire: **Ahner, Francis et al
Cabinet Régimbeau
20, rue de Chazelles
75847 Paris cedex 17 (FR)**

(56) Documents cités:
**EP-A- 0 361 874          WO-A-98/10762
US-A- 4 684 516**

EP 1 200 071 B1

**Description**

La présente invention concerne des comprimés pharmaceutiques faiblement dosés en principe actif.

L'inhomogénéité de dosage au sein des comprimés est un des problèmes majeurs rencontrés dans la formulation des compositions contenant un principe actif faiblement dosé. Pour les principes actifs de faible marge thérapeutique, le sous-dosage conduit à une inefficacité thérapeutique et le surdosage peut entraîner des effets secondaires toxiques.

Les comprimés sont constitués d'un ou plusieurs principes actifs et d'excipients de compression tels que des diluants, des liants, des lubrifiants et des délitants. Le principe actif et les excipients se présentent généralement sous la forme de poudres qui subissent une compression, avec ou sans traitement préalable.

Les différents procédés de fabrication des comprimés, à savoir la granulation par voie sèche, la compression directe et la granulation par voie humide, sont présentés dans « Remington's pharmaceutical Sciences, 16ème Ed, 1980, Mack Publ. Co. of Easton, PA, USA, pp.1553-1576 ».

La granulation par voie sèche est réservée à des productions particulières, telle que la fabrication de comprimés contenant des principes actifs hydrosolubles ou sensibles à la chaleur et à l'humidité. Cette technique est mal adaptée aux principes actifs faiblement dosés en raison de la difficulté à obtenir des mélanges homogènes de poudres sèches.

La compression directe ne comprend pas d'étape de granulation préalable à la compression et permet un gain de temps considérable. Etant donné que la plupart des principes actifs possèdent une mauvaise compressibilité et/ou sont utilisés en faible quantité par dose unitaire, ils doivent être mélangés à des excipients, qui sont directement compressibles et qui sont compatibles avec le principe actif, pour pouvoir subir une compression directe.

La compression directe est effectuée sur des machines rotatives à haute vitesse. L'appareil d'alimentation qui fonctionne en général par gravité est très sensible à l'agglomération des poudres ou à leur prise en masse. La rhéologie du mélange de poudres à comprimer est donc un facteur déterminant pour garantir l'uniformité du poids des comprimés et l'uniformité de leur contenu.

Un autre inconvénient majeur de la technique de compression directe provient du risque de séparation des poudres ou « démélange ». Ce démélange conduit à des comprimés non homogènes en composition.

Ainsi, en utilisant la technique de compression directe, on peut observer une mauvaise distribution du principe actif dans les excipients, une séparation du principe actif et des excipients pendant l'opération de mélange et surtout pendant toutes les opérations de transfert, conduisant à une variation du poids et de la teneur en principe actif des comprimés. La mauvaise fluidité du mélange de poudres est en général un facteur aggravant. La séparation du principe actif et des excipients dans le mélange de poudres avant compression est notamment observée lorsque la granulométrie du principe actif et des excipients diffèrent beaucoup.

Comme la granulation par voie sèche, la compression directe est donc mal adaptée aux comprimés faiblement dosés en principe actif.

De plus la compression directe n'est pas toujours souhaitable, notamment quand le principe actif est toxique : il est préférable de réduire l'émission de poussières en agglomérant les constituants par granulation humide.

La granulation par voie humide consiste à pulvériser sur le mélange excipients/principe(s) actif(s) en poudre un liant en solution, puis à granuler le mélange humide. La granulation par voie humide présente de nombreux avantages.

La formation des grains limite les risques de ségrégation des poudres constituée de particules de tailles et de formes différentes: l'homogénéité de la masse est ainsi mieux assurée dans le comprimé final. Par ailleurs, la transformation d'une poudre en grains permet de réduire les problèmes de poussières. L'écoulement du mélange dans la chambre de compression est facilité, ce qui assure la régularité du poids des comprimés. Enfin, la densification de la poudre permet une compression ultérieure plus aisée.

Cependant, la migration du principe actif à l'intérieur du granule peut se produire au cours de l'étape de séchage. Ce phénomène de migration est renforcé quand le principe actif est soluble dans l'excipient de granulation. Un autre problème se pose pour les principes actifs cristallins présentant un certain polymorphisme. La dissolution totale ou partielle du principe actif pendant la granulation suivie d'une précipitation pendant le séchage change la granulométrie du principe actif et éventuellement son état cristallographique. De telles modifications ont une influence directe sur la dissolution et la biodisponibilité du principe actif.

Un certain nombre d'approches ont été proposées dans l'art antérieur pour résoudre le problème de l'inhomogénéité des comprimés faiblement dosés en principe actif, comme associer à un principe actif donné un mélange particulier d'excipients, qui permet d'éviter les démélanges, microniser le principe actif, ou encore l'atomiser ou l'agglomérer avec l'excipient de compression directe.

En utilisant la granulation humide, US 3 568 828 propose de dissoudre un mélange d'oestrogène et de progestérone dans du chloroforme. Le procédé consiste ensuite à pulvériser la solution sur de la cellulose microcristalline, à sécher le mélange, à ajouter du lactose et un lubrifiant au mélange, puis à comprimer le tout. L'usage de solvants volatils représente un inconvénient majeur, pour des raisons de sécurité de fabrication et de quantités résiduelles dans les comprimés.

US 4 489 026 propose des comprimés contenant moins de 10 microgrammes de principe actif par comprimé. Ces comprimés sont obtenus en pulvérisant très lentement une solution de principe actif dans un solvant volatil sur une poudre très finement divisée d'un excipient très absorbant et insoluble dans le solvant. L'excipient est choisi parmi le lactose, l'amidon, le carbonate de calcium, le $TiO_2$ et la cellulose microcristalline. Le procédé décrit dans ce document est lent et met en oeuvre des solvants volatils.

Thiel et al. (J. Pharm. Pharmacol., 1986, 38, 335-343) ont proposé d'utiliser la technique de granulation en lit d'air fluidisé. Le principe actif est micronisé et mélangé aux excipients en poudre. Dans l'appareillage à lit d'air fluidisé, le mélange est soumis à pulvérisation avec une solution de liant.

Michael et al. (Pharmaceutical Technology, juin 1988, pp. 68-84) ont décrit un procédé qui consiste à pulvériser une solution aqueuse de PVP sur un excipient de relativement grande granulométrie, par exemple le lactose. Le principe actif de faible granulométrie est ensuite pulvérisé et se colle à la surface des particules d'excipient humidifiées. Des problèmes liés au séchage et à la mauvaise fluidité du principe actif subsistent.

WO 97/04750 décrit un procédé qui consiste à ajouter, dans un granulateur, une solution aqueuse à 1% de principe actif à un excipient directement compressible, de préférence soluble dans la solution. L'eau s'évapore sans chauffage sous l'effet d'un courant d'air. Les granulés sont ensuite comprimés. Ce procédé est limité aux principes actifs hydrosolubles.

Peu de documents de l'art antérieur décrivent la préparation de comprimés faiblement dosés par compression directe.

EP 503 521 propose de mélanger des particules de principe actif très fines à une faible quantité d'excipients, puis d'ajouter progressivement le reste d'excipients. Cette méthode repose sur l'adhésion électrostatique des fines particules de principe actif sur les particules d'excipients plus grosses. Cette méthode, très longue, ne fonctionne qu'avec certains principes actifs et dépend fortement de l'état de surface des particules de principe actif et des excipients.

Les comprimés décrits dans EP 503 521 contiennent un stéroïde micronisé et un polyol atomisé, comme le lactose, le mannitol, le sorbitol, la cellulose, le xylitol, le dextrose, le fructose ou le saccharose, de préférence le lactose. Chaque comprimé de 60 mg contient 180 microgrammes de principe actif. La variation de la teneur en principe actif est inférieure à 0,5%.

Greaves F.C. et al. (Pharmaceutical Technology, janvier 1995, pp. 60-63) et WO 95/17169 décrivent des comprimés obtenus par compression directe qui contiennent moins de 10 mg d'oestradiol micronisé. L'oestradiol est associé à du mannitol aggloméré (et non atomisé), à de la cellulose microcristalline et à du croscarmellose de sodium.

Dans le cadre de la présente invention, la Demanderesse a réussi à mettre au point un comprimé obtenu par compression directe de microgranules neutres.

La Demanderesse a en effet découvert que les microgranules neutres étaient directement compressibles.

Un excipient, pour être utilisé en compression directe, doit posséder une bonne coulabilité, ne doit pas s'agglomérer de façon spontanée, doit former un comprimé de bonne tenue mécanique ou cohésif sous l'effet d'une force de compression raisonnable et doit permettre une désintégration en un temps adapté. De nombreux diluants et liants directement compressibles ont été développés. Les excipients pour compression directe restent onéreux car ils nécessitent des procédés de préparation élaborés ou l'adjonction de nombreux additifs.

Les sucres et les carbohydrates sont couramment utilisés comme liants et délitants dans la formulation de comprimés en raison de leur goût agréable. Cependant, ils sont sous forme cristallisée et ne présentent pas toujours de bonnes propriétés de compression directe et les poudres qui en résultent sont peu fluides, si bien qu'ils doivent subir un traitement de surface ou être associés à des additifs particuliers, pour être directement compressibles.

Le lactose directement compressible est l'un des excipients parmi les plus utilisés en compression directe : il est cependant incompatible avec certains principes actifs.

L'amidon directement compressible (ou amidon prégélatinisé) subit un traitement chimique et mécanique pour éviter l'agrégation des grains d'amidon. Il est constitué de 5% amylose, 15% amylopectine et 80% amidon non modifié. Il est utilisé comme liant (sous forme d'empois), comme diluant ou comme désintégrant.

La saccharose directement compressible contient entre 95 et 98% de saccharose et un additif comme l'amidon, la maltodextrine, le sucre inverti ou un lubrifiant. Il est utilisé comme liant et surtout comme diluant.

Parmi les autres excipients pour compression directe figurent le mannitol, la cellulose microcristalline et le phosphate dicalcique. On a également développé des granulés pour compression directe présentant une bonne fluidité à base de fructose, de lactitol ou de xylitol, ils sont préparés par atomisation ou par agglomération.

Dans l'art antérieur, les microgranules neutres sont utilisés pour le montage de principe actif, et sont en général enrobés d'un film polymérique destiné à modifier la libération du principe actif.

La Pharmacopée américaine (USP XVII, 1990) décrit les microgranules neutres comme des granules essentiellement sphériques contenant entre 62,5 et 91,5% de saccharose, le reste étant essentiellement constitué d'amidon.

La pharmacopée américaine impose également une distribution de la taille des particules telle que la variation par rapport à la fourchette indiquée (par exemple 425-500, 500-600, 710-850 ou 1000-1400 microns) soit faible et que donc le diamètre des microgranules neutres soit uniforme. La solubilité des microgranules neutres varie en fonction de leur teneur en saccharose. Ils sont préparés par enrobage de saccharose cristallin avec une suspension d'amidon dans du sirop de sucre. En général, plus les microgranules neutres ont un diamètre élevé, plus la proportion d'amidon augmente. Des microgranules neutres, dont la taille est comprise entre 200 μm et 2000 μm peuvent être commercialement obtenus.

Dans l'art antérieur, de nombreux travaux de compression ont été réalisés sur des granules inertes non enrobés, mais aucune étude n'a été réalisée sur les microgranules neutres.

L'étude de la compression de noyaux préparés par extrusion sphéronisation à partir de cellulose microcristalline, de lactose ou de phosphate dicalcique, fait ressortir que la cellulose microcristalline est un matériau plastique, que le lactose se consolide par fragmentation puis par déformation plastique, et que le phosphate dicalcique dihydraté se consolide essentiellement par fragmentation. La poudre de cellulose microcristalline est connue comme étant très compressible, mais cette étude montre que les noyaux de cellulose microcristalline obtenus par extrusion sphéronisation ne sont pas compressibles et donnent des comprimés mous. Les noyaux contenant un mélange de cellulose microcristalline et de lactose sont plus compressibles et plus cassants que les noyaux de cellulose microcristalline. Enfin, les noyaux contenant un mélange de phosphate dicalcique dihydraté et de cellulose microcristalline subissent plus facilement des déformations plastiques que les deux autres types de noyaux ; ils ont un niveau de cohésion supérieur et sont plus compressibles (Schwartz JB. Nguyen NH. Schnaare RL. Compaction studies on beads : Compression and consolidation parameters, Drug Dev. Ind. Pharm, 1994, 20 (20), 3105-3129).

Des résultats similaires ont été obtenus avec des noyaux de lactose/cellulose microcristalline (Wang C. et al ., Drug Dev. Ind. Pharm., 1995, 21(7), 753-779). Ces noyaux ont en effet des propriétés de compression et de consolidation différentes de celles des poudres de même composition. La faible compressibilité des noyaux riches en cellulose microcristalline a été attribuée à la perte de plasticité de la cellulose pendant le procédé de granulation.

Les propriétés de granules contenant un mélange phosphate dicalcique/cellulose microcristalline (80/20) ont été également étudiées (Johannson B. Nicklasson F. Alderbom G., Tabletting properties of pellets of varying porosity consisting of dicalcium phosphate and microcrystalline cellulose, Pharm. Res., 1995, 12 (9), S-164).

Le mécanisme de compression de noyaux contenant de la cellulose microcristalline seule, ou en mélange avec 10% de lactose, de propanolol ou de phosphate dicalcique a été comparé à celui des poudres correspondantes. A porosités égales, les noyaux nécessitent une plus faible pression de compression que les poudres correspondantes. La compressibilité de la cellulose microcristalline diminue par addition de lactose, de phosphate dicalcique ou de propanolol (Maganti L. Celik M., Compaction studies on pellets, I. Uncoated pellets, Int. J. Pharm., 1993, 95, 29-42 ; Celik M., Compaction of multiparticulate oral dosage forms, in Multiparticulate oral drug delivery, New York, Marcel Dekker, 1994, 181-215).

Des noyaux de cellulose microcristalline contenant de la théophylline ont été préparés par extrusion sphéronisation en utilisant un mélange eau/éthanol en proportion variable. L'eau conduit à des grains plus durs et moins poreux, donc moins compressibles. Les grains préparés avec l'éthanol sont plus fragiles, se cassent pendant la compression et forment des nouvelles surfaces pour les liaisons (Millili GP, Schwartz JB, The strength of microcrystalline cellulose pellets, The effects of granulating with water ethanol mixtures, Drug Dev. Ind. Pharm., 1990, 16 (8), 1411-1426).

Il ressort de toutes les études menées sur des granules d'excipients inertes que les propriétés de compression des noyaux sont bien différentes de celles des poudres et qu'il est donc impossible de prévoir le comportement des noyaux à la compression à partir des propriétés mécaniques des poudres servant à leur élaboration .

Dans le cadre de la présente invention, la Demanderesse a mis au point un comprimé pharmaceutique faiblement dosé en principe actif.

WO 97/25028, US 4 684 516, EP 361 874 et WO 98/10762 décrivent des comprimés faiblement dosés en principe actif dans lesquels le principe actif est formulé dans des granules à libération modifiée. Ces granules sont constitués d'un noyau neutre enrobé d'une couche contenant le principe actif, puis d'une couche polymérique destinée à retarder la libération du principe actif.

Cette couche polymérique confère aux granules une compressibilité et un comportement à la compression totalement différents de ceux des granules neutres enrobés d'une seule couche de principe actif. L'enseignement de ces documents ne peut donc être appliqué aux comprimés de granules faiblement dosés en principe actif dans lesquels le principe actif est non enrobé.

La présente invention a pour objet un comprimé de microgranules faiblement dosé en principe actif contenant un diluant directement compressible, caractérisé en ce que le diluant directement compressible est constitué exclusivement de microgranules neutres, et en ce que le principe actif est monté sur les microgranules neutres et n'est pas enrobé avec un agent destiné à modifier sa libération ou à masquer son goût.

Dans le cadre de la présente invention, on entend par « microgranules neutres » des granules essentiellement sphériques contenant du saccharose et de l'amidon. Des microgranules neutres particulièrement appréciés dans le cadre de l'invention contiennent moins de 91,5 % de saccharose.

Les microgranules contenues dans les comprimés de l'invention sont constitués d'un microgranule neutre sur lequel est monté le principe actif. Etant donné que les comprimés sont faiblement dosés, il n'est pas nécessaire d'ajouter des excipients lors du montage du principe actif. Les microgranules sont de préférence constitués d'un microgranule neutre à la surface duquel est adsorbé le principe actif.

Si les excipients s'avèrent malgré tout préférables pour réaliser le montage du principe actif, le choix de leur composition et de leur proportion sera tel qu'ils ne modifient pas substantiellement les propriétés de compression des microgranules neutres.

La présente invention met avantageusement en oeuvre des particules sphériques garantissant une bonne coulabilité et une bonne homogénéité du mélange à comprimer.

Les excellentes propriétés rhéologiques des microgranules neutres en font de bons candidats comme excipient de compression directe. Le temps d'écoulement des microgranules neutres dans les conditions de test décrit à la Pharmacopée est très inférieur à 10 secondes. Cette propriété permet une alimentation très performante des machines de compression. En outre, les microgranules neutres ont un très faible volume de tassement.

Les microgranules neutres ont l'avantage de constituer un excipient de compression directe qui ne génère pas de poussières.

Enfin, les microgranules neutres ont un temps de désagrégation bien inférieur à 15 minutes.

La présente invention permet en outre d'éviter les problèmes de démélange généralement observés en compression directe, car toutes les particules à comprimer sont de la même taille.

La dimension ou la masse des comprimés est ajustable à volonté pour les faibles dosages, puisque les problèmes de démélange (qui limitent ces paramètres dans les procédés conventionnels) sont éliminés. Par ailleurs, la forme, la sécabilité et la gravure des comprimés sont respectées avec l'utilisation de tels systèmes.

Enfin, le comprimé selon l'invention peut être avantageusement utilisé comme comprimé placebo, en particulier lors d'essais techniques tels que la qualification opérationnelle d'installations de compression, la qualification de performance, les essais machines après changement de format et la validation de la régulation des machines.

Les microgranules neutres ont une taille comprise entre 100 et 2000 μm, de préférence entre 200 et 600 μm, de préférence encore entre 200 et 400 μm.

Les comprimés de la présente invention présentent une uniformité de masse très inférieure à 5 %, et de l'ordre de 1 %, pour des comprimés dont la masse est de l'ordre de 300 à 500 mg, une friabilité inférieure à 1 %, un temps de désagrégation à 37°C inférieur à 15 minutes, et une dureté de l'ordre de 0 à 20 daN. Ces paramètres sont modulables par le jeu des paramètres de compression.

Le comprimé est de préférence constitué d'un principe actif monté sur des microgranules neutres et d'excipients de compression dans une quantité inférieure à 1 % en poids par rapport au poids du comprimé

Le comprimé peut contenir en outre un lubrifiant, en une quantité inférieure à 1 % en masse, de préférence comprise entre 0,125 et 0,75 % en masse, de préférence encore de l'ordre de 0,25 % à 0,5 % en masse du comprimé.

Le lubrifiant permet la réduction des frictions interparticulaires, et entre les particules et la matrice de compression. Il permet également la réduction de l'adhérence des grains sur les poinçons et l'obtention d'une certaine brillance. Le lubrifiant est choisi par exemple parmi le stéarate de magnésium , de zinc ou de calcium, le talc, l'Aérosil®, l'acide stéarique et les PEG.

Le principe actif est avantageusement choisi parmi les stéroïdes, les neuroleptiques et autres actifs agissant sur le système nerveux central, les agents de protection du système cardio-vasculaire, les hormones, les principes actifs homéopathiques.

La quantité de principe actif est de préférence inférieure à 40 mg/g, de préférence encore inférieure à 10 mg/g, de système à comprimer, à ajuster en fonction du type de principe actif, du mode de montage et de son retentissement après montage sur les propriétés mécaniques du système prêt à être comprimé.

Le montage du principe actif sur les microgranules neutres est effectué selon des méthodes classiques, comme le montage à partir de solutions ou de suspensions, en turbine ou en lit d'air fluidisé, éventuellement en présence d'agents liants dans le solvant de pulvérisation. La quantité de liant sera adaptée en fonction de la nature et de la quantité de principe actif à monter.

Le solvant utilisé pour le montage sera en général l'eau ou tout autre solvant autorisé moyennant une étape de séchage appropriée.

Les comprimés selon l'invention peuvent être pelliculés, soit pour améliorer leur aspect, soit pour masquer la couleur, soit pour protéger le principe actif de la lumière, de l'humidité ou de l'oxygène de l'air.

Les comprimés selon l'invention peuvent également être enrobés d'un film gastrorésistant ou destiné à la libération modifiée du principe actif.

La présente invention a également pour objet un premix de compression qui consiste en une composition contenant entre 99 et 100 % en masse de microgranules neutres enrobés d'un principe actif et entre 0 et 1 % en masse d'un lubrifiant, destinée à subir une compression directe.

Le principe actif représente de préférence moins de 4 % en masse des microgranules neutres.

Enfin, la présente invention concerne un procédé de préparation des comprimés de l'invention. Selon ce procédé, la force de compression est avantageusement comprise entre 5 et 50 kN quand la surface de compression est de 1 cm$^2$ (soit 50 à 500 MPa), de préférence entre 10 et 30 kN.

La présente invention est illustrée de façon non limitative par les exemples suivants.

**<u>Exemple 1</u> : Propriétés en compression sur machine alternative des microgranules neutres non montés.**

Les microgranules neutres sont obtenus auprès de NP-Pharm.

Les propriétés sont étudiées sur trois lots A (500-600 µm), B (200-250 µm) et C (250-300 µm). Le lot A est étudié à deux niveaux de lubrification : 0,25 % (A1) et 0,5 % (A2) de stéarate de magnésium. Les lots B et C sont étudiés à un taux de lubrification de 0,25 %. On pèse 100 g de microgranules neutres de chaque lot et selon le taux de stéarate de magnésium, on ajoute 0,25 g ou 0,50 g de lubrifiant. Le mélange est réalisé au turbula (48 rpm) pendant une minute.

Chaque lot est testé à 3 niveaux de forces de compression différentes de l'ordre de : 10, 15 et 20 kN, sur une machine à comprimer alternative (Frogerais OA; poinçons 1 cm$^2$ ; hauteur de matrice standardisée à 1 cm, soit un volume utile de 1 cm$^3$).

Ces différents systèmes sont testés sur une machine à comprimer alternative équipée de capteurs de force (jauges de contraintes) et de déplacement inductif sur les poinçons supérieurs et inférieurs. Les comprimés obtenus sont soumis à un test de dureté par compression diamétrale avec une force maximum de 20 daN (type Schleuniger).

Au cours de la compression, les forces sont mesurées au niveau des deux poinçons. En tenant compte de la surface du poinçon, la force du poinçon supérieur (FPS) est traduite en pression (MPa). Le rapport des forces poinçon inférieur / poinçon supérieur donne le pourcentage de transmission.

Lors de la phase de décompression, le compact passe par une soudaine étape d'expansion liée au recouvrement élastique éventuellement suivie par un comportement viscoélastique lors de l'éjection. Cette étape peut être étudiée grâce à deux paramètres : la force résiduelle et la force d'éjection. Le contrôle de cette étape permet également de rendre compte des problèmes d'adhésion aux pièces mécaniques.

La force résiduelle est mesurée au niveau du poinçon inférieur lorsque la contrainte exercée au niveau du poinçon supérieur a cessé et que l'éjection n'est pas encore effectuée. Un optimum pour de bonnes conditions de compression des microgranules neutres est obtenu pour une valeur inférieure à 25 daN.

La force d'éjection correspond à la force nécessaire à l'éjection du comprimé hors de la matrice par le poinçon inférieur. Afin de ne pas avoir de problèmes pendant l'opération de la compression, il est couramment admis que cette force doit être inférieure ou de l'ordre de 50 daN.

De même, l'indice de cohésion égal à :

$$I_c = \frac{\text{Dureté (daN)}}{\text{Force de compression (daN)}} \times 10^5$$

est calculé.

La masse et l'épaisseur des comprimés sont également mesurées. Les résultats obtenus sont présentés tableau 1.

TABLEAU 1

| Lot | Ordre de grandeur de la de la force appliquée (kN) | FPS max. (MPa) | Pourcen-tage de tage de transmis-sion (%) | Force d'éjection (daN) | Force résiduelle (daN) | Dureté (daN) | Epais- seur (mm) | Masse (mg) | Indice De cohésion |
|-----|-----|-----|-----|-----|-----|-----|-----|-----|-----|
| A2 | 10 | 92,9 | 92,98 | 21,1 | 5,5 | 2,75 | 6,29 | 813,4 | 285 |
|  | 15 | 168,7 | 93,82 | 36,3 | 8,4 | 6,00 | 5,78 | 804,5 | 353 |
|  | 20 | 204,3 | 93,88 | 46,8 | 11,1 | 7,2 | 5,80 | 818,3 | 351 |
| A1 | 10 | 100,9 | 93,47 | 21 | 4,8 | 2,7 | 6,32 | 827,2 | 263 |
|  | 15 | 138,2 | 93,38 | 28 | 6,7 | 5,2 | 6,10 | 826,4 | 376 |
|  | 20 | 179,4 | 94,00 | 37 | 7,3 | 7,6 | 5,86 | 818,6 | 421 |
| B | 10 | 111,4 | 95,65 | 24,9 | 6 | 9 | 6,05 | 810,1 | 806 |
|  | 15 | 158,5 | 94,72 | 33 | 6,5 | 13,8 | 5,84 | 813,7 | 875 |
|  | 20 | 211,8 | 95,09 | 44 | 7,4 | ≥20 | 5,65 | 812,1 | 1000* |
| C | 10 | 118,9 | 92,66 | 20,4 | 8,9 | 7,77 | 6,06 | 832,4 | 652 |
|  | 15 | 158,8 | 95,20 | 26 | 4,9 | 12,7 | 5,78 | 820,1 | 807 |
|  | 20 | 217,2 | 95,08 | 34 | 5,7 | 18 | 5,74 | 827,9 | 830 |

* calculé sur la base d'une valeur de dureté de 20 daN.

Les résultats précédents permettent d'étudier les propriétés des microgranules neutres non montés en fonction de leur taille et du taux de lubrification.

- Comparaison entre les microgranules neutres de 500-600 μm (lot A), lubrifiés avec 0,5 % (lot A2) et 0,25 % (lot A1) de stéarate de magnésium.

L'influence du taux de stéarate de magnésium est important à évaluer car il peut retentir sur la dissolution et la libération du principe actif (caractéristiques hydrophobes du lubrifiant).

Pour les microgranules neutres de 500-600 μm, le décalage des duretés, lorsque l'on utilise 0,25 % ou 0,5 % de stéarate de magnésium, est très faible.

Le pourcentage de transmission, aussi appelé « l'indice de lubrification », reste pour les deux systèmes très élevé, aux alentours de 93 %.

Enfin, le rapport entre force de poinçon supérieur (FPS) et force à l'éjection évolue de façon linéaire avec un coefficient de corrélation de 0,99, et laisse donc estimer (par extrapolation) que la limite acceptable de la force à l'éjection de 50 daN sera atteinte pour les deux lots A1 et A2 entre 230-260 MPa.

Sur la base de ces résultats, il semble qu'un taux de stéarate de magnésium de 0,25 % soit suffisant.

- Comparaison entre les microgranules neutres de différentes tailles (lots A1, B et C) avec une lubrification à 0,25 % de stéarate de magnésium.

La dureté décroît de façon très sensible lorsque la taille des microgranules augmente. A niveaux identiques de force croissante, la dureté croît de façon plus rapide avec les systèmes plus petits.

La force à l'éjection est la plus faible pour le lot C. Les deux autres lots A1 et B sont pratiquement identiques, avec une force d'éjection légèrement plus élevée. La limite d'acceptabilité de 50 daN serait atteinte aux alentours d'une pression appliquée de 250 MPa pour A1 et B, et dépasserait 300 MPa pour C (évaluation par extrapollation).

On note clairement que l'indice de cohésion décroît avec l'augmentation de la taille des microgranules. L'indice de cohésion optimal d'un excipient pour compression directe se trouve aux alentours de 1000, cette valeur se trouve atteinte ou pratiquement atteinte pour les microgranules neutres de petite dimension testé (lot B).

- Conclusion

Pour le système des neutres de 500-600 μm un taux de lubrification à 0,25 % semble suffisant pour obtenir une machinabilité suffisante avec une gamme de dureté satisfaisante. La limite de machinabilité concernant la force à l'éjection serait atteinte aux alentours d'une pression de compression de 230-260 MPa. Le rapport de transmission toujours supérieur à 90 % est excellent dans tous les cas de figure.

Au fur et à mesure que la taille des microgranules diminue, la comprimabilité est plus élevée. L'indice de cohésion d'environ 1000 (regardé comme valeur d'excellence) est pratiquement atteint avec des microgranules d'environ 200-300 μm. Les propriétés de cohésion des systèmes non montés de dimension faible à intermédiaire sont néanmoins très bonnes.

**Exemple 2 : Propriétés en compression des microgranules neutres montés avec la Molsidomine, sur machine à comprimer alternative instrumentée.**

On prépare les lots de microgranules neutres montés suivants, en utilisant des microgranules neutres de granulométrie comprise entre 200 et 300 μm. Le taux de lubrification est fixé à 0,25 %.

Le principe actif est la Molsidomine, montée sur les neutres à partir d'une solution aqueuse ou en présence d'un liant en solution, le Pharmacoat 603, selon les rapports quantitatifs donnés dans le tableau suivant :

| LOT | TAUX DE MONTAGE | % DE LIANT |
|-----|-----------------|------------|
| Ma  | 6,3 mg/g        | -          |
| Mb  | 11,4 mg/g       | -          |
| Mc  | 5,9 mg/g        | 0,14%      |
| Md  | 11,2 mg/g       | 0,27 %     |

On procède comme dans l'exemple 1 et les lots sont listés à trois niveaux de force de compression entre 7,5 et 26 kN (soit entre 75 et 260 MPa exprimé en contrainte).

Les comprimés sont soumis à un test de dureté comme dans l'exemple 1, leur masse est également mesurée. Les résultats sont présentés dans le tableau 2.

## TABLEAU 2

| LOT | Ordre de grandeur de la Force Appliqueé (kN) | FPS max (MPa) | Pourcentage de transmission | Force d'éjection (daN) | Force résiduelle (daN) | Dureté (daN) | Masse (mg) | Indice de cohésion |
|---|---|---|---|---|---|---|---|---|
| Neutres | 7 | $75 \pm 2$ | $93,4 \pm 0,4$ | $17 \pm 1$ | $5,3 \pm 0,5$ | $5,8 \pm 0,4$ | $839 \pm 11$ | $776 \pm 39$ |
| (200-300µm) | 12 | $120 \pm 2$ | $94,5 \pm 0,1$ | $24 \pm 1$ | $8\,0 \pm 0,0$ | $11,0 \pm 0,3$ | $842 \pm 2$ | $900 \pm 24$ |
| | 18 | $175 \pm 3$ | $94,8 \pm 0,1$ | $32 \pm 2$ | $8,6 \pm 0,5$ | $17,3 \pm 0,7$ | $840 \pm 1$ | $988 \pm 34$ |
| Ma | 18 | $173 \pm 7$ | $94,3 \pm 0,2$ | $45 \pm 2$ | $8,2 \pm 0,6$ | $6,4 \pm 0,3$ | $783 \pm 5$ | $370 \pm 11$ |
| | 25 | $240 \pm 12$ | $94,9 \pm 0,3$ | $59 \pm 5$ | $11,6 \pm 0,8$ | $10,2 \pm 0,5$ | $795 \pm 5$ | $425 \pm 14$ |
| | 28 | $272 \pm 10$ | $94,6 \pm 0,1$ | $73 \pm 7$ | $15,0 \pm 0,0$ | $10,9 \pm 0,4$ | $806 \pm 4$ | $402 \pm 17$ |
| Mb | 15 | $152 \pm 13$ | $94,1 \pm 0,2$ | $40 \pm 4$ | $9 \pm 1$ | $7 \pm 1$ | $806 \pm 10$ | $448 \pm 26$ |
| | 20 | $205 \pm 18$ | $94,2 \pm 0,2$ | $54 \pm 5$ | $12 \pm 1$ | $10 \pm 1$ | $813 \pm 11$ | $480 \pm 32$ |
| | 26 | $262 \pm 11$ | $94,3 \pm 0,1$ | $71 \pm 3$ | $16 \pm 1$ | $13,0 \pm 0,6$ | $819 \pm 5$ | $495 \pm 16$ |
| Mc | 14 | $138 \pm 8$ | $94,8 \pm 0,2$ | $32 \pm 2$ | $2 \pm 2$ | $5,7 \pm 0,5$ | $755 \pm 6$ | $416 \pm 20$ |
| | 20 | $205 \pm 8$ | $95,2 \pm 0,2$ | $47 \pm 3$ | $11 \pm 1$ | $12,0 \pm 0,8$ | $765 \pm 4$ | $585 \pm 30$ |
| | 25 | $255 \pm 6$ | $95,5 \pm 0,2$ | $60 \pm 2$ | $12 \pm 0$ | $14,2 \pm 0,8$ | $759 \pm 2$ | $557 \pm 25$ |
| Md | 11 | $111 \pm 2$ | $94,4 \pm 0,2$ | $26,7 \pm 0,8$ | $4,6 \pm 0,5$ | $5,0 \pm 0,2$ | $756 \pm 2$ | $453 \pm 17$ |
| | 17 | $175 \pm 5$ | $94,8 \pm 0,1$ | $41 \pm 2$ | $8,0 \pm 0,0$ | $9,0 \pm 0,4$ | $769 \pm 3$ | $512 \pm 21$ |
| | 25 | $252 \pm 6$ | $95,0 \pm 0,2$ | $58 \pm 4$ | $10,7 \pm 0,8$ | $15,0 \pm 0,5$ | $778 \pm 2$ | $594 \pm 19$ |

Les comprimés obtenus à partir de neutres montés en PA sont comparés aux comprimés obtenus à partir des mêmes neutres (même dimension), mais non montés en principe actif. Ces essais permettent donc d'étudier l'influence du montage des microgranules neutres avec la Molsidomine sur leurs propriétés en compression.

Pour les neutres non montés, on retrouve ce qui a été décrit dans l'exemple 1 pour les systèmes de granulométrie voisine.

Les duretés obtenues sont satisfaisantes pour des forces de compression relativement faibles (75 MPa) et elles augmentent rapidement quand la force de compression augmente. Des duretés importantes de 17 daN sont obtenues pour des forces de compression encore relativement faibles de l'ordre de 18 kN soit 180 MPa. Les indices de cohésion tendent vers l'excellence à des valeurs de l'ordre de 900.

Pour les systèmes montés, les niveaux de dureté sont plus faibles à force de compression identique. Cependant, ces niveaux de dureté sont très satisfaisants pour des forces de compression de l'ordre de 15 à 25 kN. Les indices de cohésion sont plus faibles que pour les neutres non montés, mais restent à des valeurs très acceptables de l'ordre de 400 à 500. L'influence de la quantité de principe actif montée n'est pas très sensible sur ces systèmes. Par contre, il est possible de noter l'influence du procédé de montage, puisque les systèmes montés en présence d'un liant s'avèrent plus cohésifs que les systèmes montés à partir d'une solution simple.

Dans tous les cas, les forces résiduelles après compression sont très faibles et toujours inférieures à 15 daN pour les forces de compression testées.

Les forces d'éjection sont en général acceptables, mais tendent vers les valeurs limites de 50 à 60 daN quand les forces de compression augmentent à 25 kN.

Aucun phénomène de collage, grippage ou de décalotage des comprimés formés n'est cependant noté. Une lubrification très légèrement supérieure permet de diminuer les forces à l'éjection aux plus hautes pressions. La marge de manoeuvre, dans le jeu classique formulation/fabrication (couple produit/machine) reste ici très importante.

Les rapports de transmission sont dans tous les cas excellents, de l'ordre de 93 à 95 %.

La masse des comprimés est très stable, avec une variation aléatoire autour de la moyenne inférieure à 1,5 % dans le pire des cas.

Conclusion

Les systèmes de microgranules neutres montés en principe actif présentent des propriétés de compression très intéressantes. La cohésion de ces systèmes est très bonne, même si elle est influencée par la présence de l'actif monté à la surface des neutres.

La transmission de force, et la régularité des masses constituent deux atouts majeurs de ces systèmes.

**Exemple 3 : propriétés des microgranules neutres non montés sur machine rotative en fonction du taux de lubrifiant.**

Cet essai consiste en la compression sur machine à comprimer rotative de microgranules neutres non montés, à différents taux de stéarate de magnésium afin de déterminer le taux minimal de lubrifiant nécessaire pour obtenir des comprimés de caractéristiques satisfaisantes.

Les taux de stéarate de magnésium étudiés sont 0,125, 0,25, 0,5 et 0,75 %.

La taille des microgranules neutres est comprise entre 315 et 400 $\mu$m.

Un pré-mélange est effectué entre la moitié de la masse des microgranules et la moitié de la masse de stéarate à l'aide du mélangeur TURBULA pendant 1 minutes.

Ensuite, la partie pré-mélangée ainsi que le reste des microgranules et du stéarate sont mélangés au mélangeur cubique ERWEKA pendant 5 minutes.

Le volume de la matrice est réglé afin d'obtenir des comprimés de l'ordre de 350 mg. La compression est réglée afin que la dureté des compacts ait une valeur acceptable pour chaque teneur en stéarate. Le réglage de la pré-compression est indicé à 4 et non modifié.

Après optimisation des réglages de masse et de dureté, et après 30 à 60 secondes de fonctionnement, 20 comprimés sont prélevés toutes les 30 secondes pendant 5 minutes.

Les quatre paramètres de dureté, masse, fiabilité et temps de désagrégation sont ensuite mesurés.

- **Dureté:** cet essai est destiné à déterminer, dans des conditions définies, la résistance à la rupture des comprimés, mesurée par la force nécessaire pour provoquer leur rupture par compression diamétrale. Elle est mesurée à l'aide de l'appareil ERWEKA et effectuée sur 10 comprimés.
- **Masse:** elle est mesurée à l'aide d'une balance SARTORIUS et effectuée sur 10 comprimés.
- **Friabilité :** cet essai est destiné à déterminer, dans des conditions définies, la friabilité des comprimés non enrobés, c'est-à-dire le phénomène par lequel la surface des comprimés est endommagée ou présente des signes d'abrasion ou de rupture sous l'effet de chocs mécaniques ou d'une attrition.
Elle est mesurée à l'aide de l'appareil ERWEKA et effectuée sur 10 comprimés.

- **Temps de désagrégation** : il est destiné à déterminer la plus ou moins grande aptitude des comprimés à se dissoudre dans le temps en milieu liquide. Il est mesuré à l'aide de l'appareil ERWEKA et effectué dans l'eau à 37°C, sur 6 comprimés.
- Les résultats obtenus sont présentés dans les tableaux 3 et 4.

## TABLEAU 3

| Stéarate de magnésium (%) | Temps (s) | 30 | 60 | 90 | 120 | 150 | 180 | 210 | 240 | 270 | 300 |
|---|---|---|---|---|---|---|---|---|---|---|---|
| 0,125 | Masse (mg) | 358.5 | 354,7 | 354,8 | 355 | 365 | 352,2 | 354,8 | 356,4 | 358,2 | 353,9 |
| | Dureté (N) | 84.9 | 92,9 | 85,5 | 81,6 | 82,9 | 74,3 | 93,8 | 85,1 | 78,5 | 83,4 |
| | Friabilité (%) | 0,85 | 0,22 | 0,14 | 0,1 | 0,12 | 0,12 | 0,10 | 0,15 | 0,20 | 0,19 |
| 0,25 | Masse (mg) | 357,5 | 357 | 355,7 | 350,5 | 361,7 | 353,9 | 360,3 | 352,9 | 361,5 | 357,5 |
| | Dureté (N) | 87 | 83 | 82 | 88 | 82 | 89 | 85,3 | 81,8 | 83 | 88,6 |
| | Friabilité (%) | 0,06 | 0,11 | 0,19 | 0,44 | 0,88 | 0,59 | 0,13 | 0,13 | 0,19 | 0,23 |
| 0,5 | Masse (mg) | 343,2 | 345,6 | 346,9 | 348,8 | 345,6 | 351 | 354,2 | 350,6 | 359,5 | 346,3 |
| | Dureté (N) | 70,3 | 70,3 | 71,4 | 68,3 | 71,2 | 65,5 | 68,6 | 69,3 | 75,5 | 74,1 |
| | Friabilité (%) | 0,158 | 0,176 | 0,179 | 0,098 | 0,088 | 0,135 | 0,014 | 0,131 | 0,115 | 0,138 |
| 0,75 | Masse (mg) | 349,3 | 345,5 | 354,7 | 351,4 | 352,1 | 351,4 | 345,8 | 351,4 | 350,8 | 353,7 |
| | Dureté (N) | 59 | 59,5 | 56,8 | 56,1 | 56,9 | 60 | 58,4 | 56 | 61,1 | 59,9 |
| | Friabilité (%) | 0,1 | 0,1 | 0,12 | 0,36 | 0,09 | 0,51 | 0,16 | 0,06 | 0,01 | 0,13 |

TABLEAU 4

| % de stéarate de magnésium | Temps de désagrégation (minutes) |
|---|---|
| 0,125 | 8,8 |
| 0,25 | 9,5 |
| 0,50 | 11 |
| 0,75 | 22 |

**Conclusion**

- **Masse** : la Pharmacopée Française préconise un écart limite en pourcentage de la masse moyenne de 5 % pour des comprimés correspondant à la masse engagée. Pour les différents taux de stéarate de magnésium, les valeurs fluctuent de part et d'autres de la moyenne donc la distribution est aléatoire. Tous les lots sont conformes car les valeurs trouvées sont très inférieures aux limites imposées par la Pharmacopée.
- **Friabilité** : d'après la Pharmacopée Française, la perte de masse maximale considérée comme acceptable est de 1 % de la masse des comprimés soumis à l'essai. Pour les différents taux de stéarate de magnésium utilisés, nous nous rendons compte que les résultats sont très inférieurs à la norme de la Pharmacopée donc les différents lots sont conformes.
- **Dureté** : la Pharmacopée Française n'impose pas de limite. Pour tous les taux de stéarate de magnésium, les résultats montrent que nous sommes toujours dans les limites de réglage donc les différents lots sont conformes.
- **Temps de désagrégation :** la Pharmacopée Française fixe des conditions de délitement en fonction du type de comprimé. Pour les comprimés nus ou non enrobés, le temps doit être inférieur à 15 minutes. Nos résultats sont donc conformes à la norme de la Pharmacopée, sauf à 0,75 % de stéarate de magnésium et à la force de compression utilisée où le temps est de 22 minutes.

La machinabilité des microgranules neutres sur machine à comprimer rotative est donc démontrée, avec d'excellents résultats. Il est à noter que les rendements de production sont excellents : la trémie se vide sans aide extérieure jusqu'aux derniers grains. L'absence d'empoussièrement dans la machine et l'atmosphère pendant toute l'opération est également très appréciable.

**Revendications**

1. Comprimé de microgranules faiblement dosé en principe actif contenant un diluant directement compressible, **caractérisé en ce que** le diluant directement compressible est constitué exclusivement de microgranules neutres, et **en ce que** le principe actif est monté sur les microgranules neutres et n'est pas enrobé avec un agent destiné à modifier sa libération ou à masquer son goût.

2. Comprimé selon la revendication 1, **caractérisé en ce que** la taille des microgranules neutres est comprise entre 100 et 2000 μm, de préférence entre 200 et 600 μm.

3. Comprimé selon la revendication 2, **caractérisé en ce que** la taille des microgranules neutres est comprise entre 200 et 400 μm.

4. Comprimé selon l'une des revendications précédentes, **caractérisé en ce que** sa dureté est comprise entre 0 et 20 daN.

5. Comprimé selon l'une des revendications précédentes, **caractérisé en ce que** sa friabilité est comprise entre 0 et 1 %.

6. Comprimé selon l'une des revendications précédentes, **caractérisé en ce que** son temps de délitement est inférieur à 15 minutes.

7. Comprimé selon l'une des revendications précédentes, **caractérisé en ce qu'**il est constitué d'un principe actif monté sur des microgranules neutres et d'excipients de compression dans une quantité inférieure à 1 % en poids par rapport au poids du comprimé.

**8.** Comprimé selon la revendication 7, **caractérisé en ce qu'**il contient en outre un lubrifiant dans une quantité inférieure à 1 % en masse du comprimé.

**9.** Comprimé selon la revendication 8, **caractérisé en ce que** la teneur en lubrifiant est comprise entre 0,125 et 0,75 % en masse, de préférence 0,25 % en masse.

**10.** Comprimé selon l'une des revendications précédentes, **caractérisé en ce que** la quantité de principe actif est inférieure à 40 mg/g de système à comprimer, de préférence inférieure à 10 mg/g.

**11.** Premix de compression pour la préparation du comprimé selon l'une des revendications 1 à 10, contenant

- entre 99 et 100 % en masse de microgranules neutres sur lesquelles est monté un principe actif, et
- entre 0 et 1 % en masse d'un lubrifiant, destiné à subir une compression directe.

**12.** Premix selon la revendication 11, **caractérisé en ce que** le principe actif monté sur les microgranules neutres représente moins de 4 % en masse des microgranules neutres.

**13.** Procédé de préparation du comprimé selon l'une des revendications 1 à 10, **caractérisé en ce qu'**il est obtenu par compression directe d'un premix selon l'une des revendications 11 et 12, par mise en oeuvre d'une force de compression comprise entre 5 et 50 kN, de préférence entre 10 et 30 kN.

**Claims**

**1.** Tablet comprising a low dose of active principle formed from microgranules comprising a directly compressible diluent, **characterized in that** the directly compressible diluent is composed solely of neutral microgranules and **in that** the active principle is attached as a coating to the neutral microgranules and is not coated with an agent intended to modify its release or to mask its taste.

**2.** Tablet according to Claim 1, **characterized in that** the size of the neutral microgranules is between 100 and 2000 µm, preferably between 200 and 600 µm.

**3.** Tablet according to Claim 2, **characterized in that** the size of the neutral microgranules is between 200 and 400 µm.

**4.** Tablet according to one of the preceding claims, **characterized in that** its hardness is between 0 and 20 daN.

**5.** Tablet according to one of the preceding claims, **characterized in that** its friability is between 0 and 1%.

**6.** Tablet according to one of the preceding claims, **characterized in that** its disintegration time is less than 15 minutes.

**7.** Tablet according to one of the preceding claims, **characterized in that** it is composed of an active principle attached as a coating to neutral microgranules and of compression excipients in an amount of less than 1% by weight with respect to the weight of the tablet.

**8.** Tablet according to Claim 7, **characterized in that** it additionally comprises a lubricant in an amount of less than 1% by mass of the tablet.

**9.** Tablet according to Claim 8, **characterized in that** the content of lubricant is between 0.125 and 0.75% by mass, preferably 0.25% by mass.

**10.** Tablet according to one of the preceding claims, **characterized in that** the amount of active principle is less than 40 mg/g of system to be tableted, preferably less than 10 mg/g.

**11.** Compression premix for the preparation of the tablet according to one of Claims 1 to 10, containing

- between 99 and 100% by mass of neutral microgranules to which is attached a coating of an active principle, and
- between 0 and 1% by mass of a lubricant,

which premix is intended to be subject to direct compression.

12. Premix according to Claim 11, **characterized in that** the active principle attached as a coating to the neutral microgranules represents less than 4% by mass of the neutral microgranules.

13. Process for the preparation of the tablet according to one of Claims 1 to 10, **characterized in that** it is obtained by direct compression of the premix according to either of Claims 11 and 12 by employing a compression force of between 5 and 50 kN, preferably between 10 and 30 kN.

**Patentansprüche**

1. Tablette von Mikrokörnern, die an Wirkstoff niedrig dosiert ist, enthaltend ein direkt verdichtbares Verdünnungsmittel, **dadurch gekennzeichnet, dass** das direkt verdichtbare Verdünnungsmittel ausschließlich aus neutralen Mikrokörnern gebildet wird und dass der Wirkstoff auf die neutralen Mikrokörner aufgebracht ist und nicht mit einem Mittel, das dazu bestimmt ist, dessen Freisetzung zu modifizieren oder seinen Geschmack zu maskieren, umhüllt ist.

2. Tablette nach Anspruch 1, **dadurch gekennzeichnet, dass** die Größe der neutralen Mikrokörner zwischen 100 und 2000 µm, vorzugsweise zwischen 200 und 600 µm liegt. ,

3. Tablette nach Anspruch 2, **dadurch gekennzeichnet, dass** die Größe der neutralen Mikrokörner zwischen 200 und 400 µm liegt.

4. Tablette nach einem der vorangegangenen Ansprüche, **dadurch gekennzeichnet, dass** ihre Härte zwischen 0 und 20 daN liegt.

5. Tablette nach einem der vorangegangenen Ansprüche, **dadurch gekennzeichnet, dass** ihre Mürbheit zwischen 0 und 1% liegt.

6. Tablette nach einem der vorangegangenen Ansprüche, **dadurch gekennzeichnet, dass** ihre Zerfallszeit unter 15 min liegt.

7. Tablette nach einem der vorangegangenen Ansprüche, **dadurch gekennzeichnet, dass** sie aus einem auf neutrale Mikrokörner aufgebrachten Wirkstoff und Verdichtungsträgersubstanzen in einer Menge unter 1 Gew.-% bezogen auf das Gewicht der Tablette gebildet wird.

8. Tablette nach Anspruch 7, **dadurch gekennzeichnet, dass** sie außerdem ein Gleitmittel in einer Menge unter 1 Masseprozent der Tablette enthält.

9. Tablette nach Anspruch 8, **dadurch gekennzeichnet, dass** der Gehalt an Gleitmittel zwischen 0,125 und 0,75 Masseprozent vorzugsweise 0,25 Masseprozent liegt.

10. Tablette nach einem der vorangegangenen Ansprüche, **dadurch gekennzeichnet, dass** die Wirkstoffmenge unter 40 mg/g von zu verdichtendem System, vorzugsweise unter 10 mg/g liegt.

11. Verdichtungsvormischung für die Herstellung der Tablette nach einem der Ansprüche 1 bis 10 enthaltend

- zwischen 99 und 100 Masseprozent neutrale Mikrokörner, auf die ein Wirkstoff aufgebracht ist, und
- zwischen 0 und 1 Masseprozent eines Gleitmittels, die dazu bestimmt ist, eine direkte Verdichtung zu durchlaufen.

12. Vormischung nach Anspruch 11, **dadurch gekennzeichnet, dass** der auf die neutralen Mikrokörner aufgebrachte Wirkstoff weniger als 4 Masseprozent der neutralen Mikrokörner darstellt.

13. Verfahren zur Herstellung der Tablette nach einem der Ansprüche 1 bis 10, **dadurch gekennzeichnet, dass** sie durch direkte Verdichtung einer Vormischung nach einem der Ansprüche 11 und 12 durch Ausüben einer Verdichtungskraft zwischen 5 und 50 kN, vorzugsweise zwischen 10 und 30 kN erhalten wird.